# EUROPEAN PATENT SPECIFICATION

(11) **EP 1 948 261 B1**
(45) Date of publication and mention of the grant of the patent: **10.11.2010**
(21) Application number: 06826165.0
(22) Date of filing: 13.10.2006
(51) Int. Cl.: A61L 27/36, A61L 27/50, A61L 31/00, A61L 31/14

(54) **MEDICAL DEVICE WITH AFFIXATION MEANS**
MEDIZINPRODUKT MIT FIXIERMITTELN
DISPOSITIF MEDICAL COMPRENANT DES DISPOSITIFS DE FIXATION

(30) Priority: 18.10.2005 US 727912 P
(43) Date of publication of application: 30.07.2008
(73) Proprietor: Cook Biotech Incorporated, West Lafayette, IN 47906 (US)
(72) Inventor: MCALEXANDER, Chad, S., Delphi, IN 46923 (US); HILES, Michael, C., Lafayette, IN 47905-9472 (US)
(74) Representative: Garratt, Peter Douglas
(86) International application number: PCT/US2006/040681
(87) International publication number: WO 2007/047743

(56) References cited:
- WO-A-98/35632
- WO-A-99/63051
- GB-A- 1 352 282
- US-A1- 2003 041 426
- US-A1- 2004 141 956
- US-A1- 2005 085 924
- US-B1- 6 371 985
- US-B1- 6 746 458

## Description

### FIELD OF THE INVENTION

The present invention relates to the field of medical devices. More particularly, it relates to medical devices that are implanted in or attached to a living organism.

### BACKGROUND OF THE INVENTION

Medical devices, including for example, drug pump, pacemakers, patching structures, collared heart valves, and port devices (e.g., Cook Vital-Port® available from Cook Vascular Corporation, Leechburg, Pennsylvania) are commonly implanted into or attached to the body of a patient to treat a medical condition. In most procedures for device placement, the device is secured with sutures. Currently available devices commonly have a fabric or ring structure(s) through which sutures may be secured. Alignment and placement of sutures on, in, or through such structures is another step of procedures that are commonly complex and time-consuming.

As one example of medical devices, hernia repair devices typically consist of sheets of material that are either woven from filaments or constructed from components extruded as a sheet. Such a device is introduced to a body cavity, usually the abdominal cavity, through an open incision or through a trocar in a laparoscopic surgical procedure.
Laparoscopic introduction is preferable as minimally invasive surgical techniques minimize patient trauma and recovery time. After being introduced, the hernia repair device is typically pressed up against the inner surface of the body cavity (e.g., in repairing a ventral abdominal hernia, the device is placed against the inner surface of the peritoneum). Tacks and/or sutures are then employed to affix the device to the inner surface of the body cavity (e.g., by securing the patch to the abdominal muscles). This is most commonly accomplished by pushing the sutures or tacks through the inner surface of the device into the fascia lining the body cavity. Particularly in laparoscopic surgery, the affixation process requires extra tools and can impose extra burdens on the surgeon effecting the hernia repair. Additionally, a surface of the affixation means (e.g. tack, suture) is left exposed to the inside of the body cavity, resulting in potential complications. Such complications include abrasion of viscera within the body cavity resulting in inflammation and patient pain. Other, or further, potential complications include adhesions forming between the affixation means and the viscera. This adhesion of the viscera to structures attached to the body wall can possibly result in lethal complications, such as bowel ischemia, ileus, and necrosis.

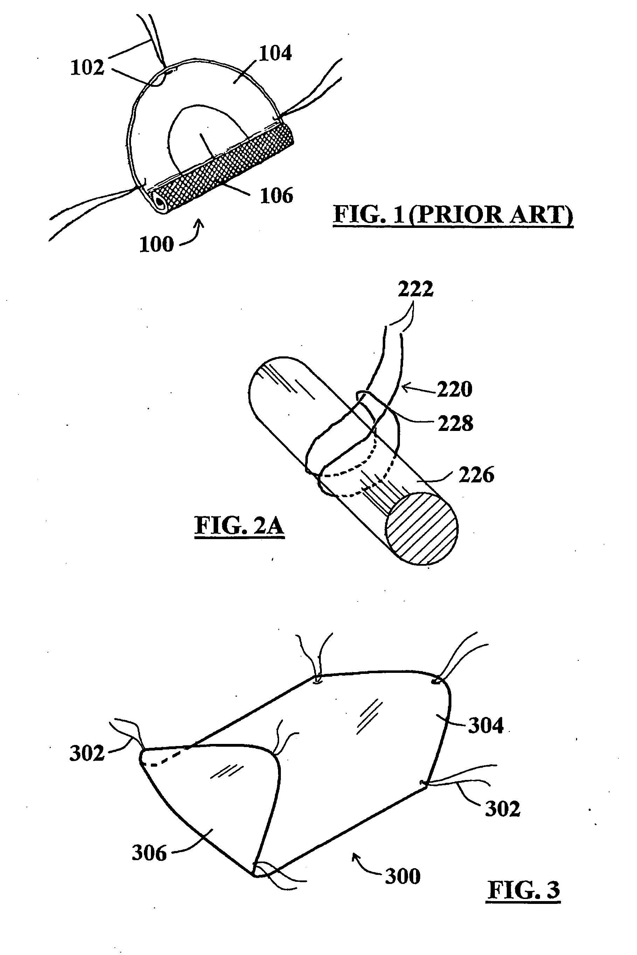

FIG. 1 shows an example of a prior art hernia patch 100 with sutures 102 disposed thereon, partially rolled up with the bottom side 104 rolled to the inside. In a typical hernia repair procedure, the sutures 102 are mounted by a user from the bottom side 104 through to the top side 106 of the patch 100 or are looped completely through both layers beginning from and returning to the top side 106. The typical patch has a smooth bottom side 104 comprising, for example, polytetrafluoroethylene and/or expanded polytetrafluoroethylene (PTFE or ePTFE) and a textured top side 106 comprising, for example, polypropylene mesh. In an application of the illustrated patch device 100, the top side 106 is directed toward the exterior of the patient and is placed in contact with a mounting surface (e.g., the peritoneal membrane), while the bottom side 104 faces the patient's interior. As mentioned above, the portion of the suture 102 that is exposed on the bottom side 104 provides a site where adhesion with underlying viscera (e.g., in the repair of a ventral hernia) is known to be more likely to occur than on other areas of the patch.

Some suture-anchoring devices incorporating pre-attached sutures are known in the prior art, but pre-attachment of sutures has not been systematically applied to implantable and other medical devices, in a manner that minimizes the likelihood of adhesions to the sutures.

What is needed therefore is a medical device suitable for insertion into a patient, such as a hernia repair device or a graft, that provides integrated or otherwise pre-placed convenient-to-use affixation means without leaving exposed surfaces of the affixation means to potentially cause post-operative damage to nearby tissues including viscera.

### BRIEF SUMMARY OF THE INVENTION

The present invention provides a general approach to the design of medical devices that are implanted in a patient, where affixation means are pre-attached such that they are less likely to be associated with deleterious tissue adhesion. Medical devices of the present invention preferably include configurations that sequester the affixation means, e.g., a suture, on at least one side of the medical device from exposure to a patient's viscera or other adjacent/ proximate tissues that are preferably not intended for attachment (e.g., adhesion) to the device. In addition, the pre-attachment aspect of the present invention reduces the steps to be completed by a physician placing such a device, and renders the placement easier and faster in contrast to placing the same device with no pre-attached suture(s).

In one aspect, the present invention provides a class of medical devices that include pre-attached sutures for attachment to or implantation into a body. In preferred embodiments of this class of medical devices, the sutures are positioned and/or oriented to reduce the likelihood of adhesions forming between the sutures and designated nearby tissue and/or viscera.

In another aspect, the present invention provides a medical device suitable for tissue repair and including convenient-to-use integrated affixation means not having exposed surfaces of the affixation means to potentially cause post-operative damage to the viscera and/or other proximate tissue(s) in some embodiments the device may be configured for repair of ventral or other hernias and is also suitable for repairing other injuries or conditions requiring tissue openings or wounds to be patched, having integral affixation means that are not exposed to the non-affixed side of the device. The integral nature of the affixation means also provides an advantage of convenience in use. The preferred affixation means include sutures or tacks, but those of skill in the art will appreciate that other currently existing or future-developed affixation means are appropriate for use within the scope of the present invention.

More particularly, the present invention is described in the appended claims.

### BRIEF DESCRIPTION OF SEVERAL VIEWS OF THE DRAWINGS

FIG. 1 is a perspective view of a prior art hernia patch with sutures disposed thereon;

FIG. 2 is a longitudinal cross-sectional view of a multilayered tissue repair device embodiment having sutures disposed therein.

FIG. 2A is a perspective view illustrating a girth hitch;

FIG. 3 is a perspective illustration of a of a tissue repair device illustrating one configuration of pre-placed sutures;

FIG. 4 is a perspective illustration of an implantable venous access port device with pre-attached sutures;

FIG. 5 depicts an implantable drug pump with pre-attached sutures; and

FIG. 6 shows an implantable aortic valve device with pre-attached sutures.

### DETAILED DESCRIPTION OF THE INVENTION

In one aspect, the present invention includes a medical device having a pre-attached affixation means, wherein the affixation means is asymmetrically disposed on the medical device. The affixation means extends preferably from one or more surfaces of the medical device, thereby providing the means to draw that surface or surfaces of the medical device close to a surface or surfaces within the patient's body. The surface or surfaces of the medical device of the present invention that are designed to be affixed directly to a tissue, such as a site of treatment in or on a patient, are referred to herein as the proximal surface or surfaces. Other surfaces of the medical device that are designed to not be directly affixed to any tissue in or on a patient are referred to herein as the distal surface or surfaces. The affixation means can include, for example, a suture or other attachment structure (e.g., flanged anchor, staple, ferromagnetic affixation structure) that extends from a proximal surface of the medical device and serves to secure and hold fast that face of the medical device to tissue that is on or within the body. Within the scope of the present invention, "affixation means" is intended to include any structure now in use or developed in the future that is used to affix a medical device in or on a patient. Further contributing to the asymmetric nature of the medical device with respect to the affixation means, the affixation means preferably has no other point protrusion from the medical device apart from that extending out on the face designed to preferably be in contact with a tissue of the patient (i.e., in a preferred embodiment, the affixation means extends out from the proximal surface(s) of the medical device, but not from any of its other surfaces).

In another aspect, the present invention includes a medical device with one or more first layers comprising a first biocompatible material; one or more second layers comprising a second biocompatible material; and one or more sutures disposed on the first layer(s), the second layer(s), or both. The medical device includes one or more proximal sides and one or more distal sides, which proximal and distal sides relate to one or more tissue surfaces to which the medical device is sutured when employed in treating a patient. Specifically, with reference to the terminology herein when a device of the present invention is employed in treating a patient, the proximal side(s) refer to those portions of the device that are preferably adjacent to and/or in contact with one or more tissue surfaces to which the device may or should adhere (e.g., by tissue in-growth/ incorporation). In contrast, the distal side(s) refer those portions of the device that may be adjacent to and/or in contact with one or more tissue surfaces to which the device most preferably will not adhere. Preferably, the one or more sutures are disposed such that at least one end of each suture extends from the one or more proximal sides and substantially none of the one or more sutures is exposed on the one or more distal sides.

Presented herein is a series of preferred embodiments of the present invention. While reference is made to tissues included herewith, the particular embodiments so illustrated and described should not limit the scope of the present invention. Those skilled in the art will readily perceive alternative materials and designs usefully employed in the context of the invention disclosed here.

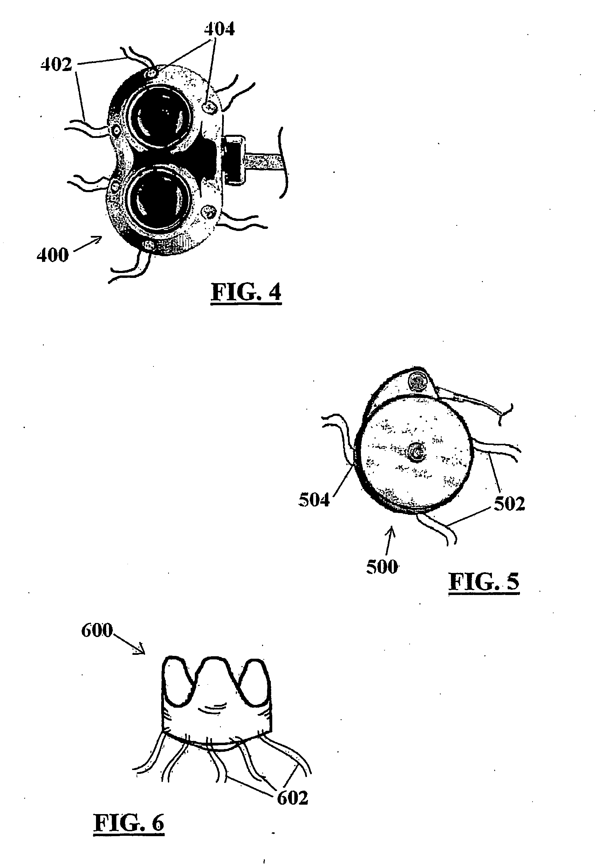

FIGS. 4-6 illustrate general embodiments of the present invention, including examples of devices with pre-attached sutures (the sutures are not drawn to scale, and alternatively may be embodied as barbed sutures). FIG. 4 illustrates an implantable venous access port device 400 including pre-attached sutures 402. The suture attachment sites 404 include a biocompatible material (e.g., silicone or PTFE) presenting a generally smooth surface and at least partially covering the sutures 402 to lessen the likelihood that nearby tissue will ingrow or otherwise adversely adhere to the venous access port device 400. FIG. 5 depicts an implantable drug pump 500 with pre-attached sutures 502. The suture attachment sites 504 include a biocompatible material presenting a generally smooth surface and at least partially covering the sutures 502 to lessen the likelihood that nearby tissue will ingrow or otherwise adhere to a portion of the implantable drug pump 500 that is not desirable. FIG. 6 shows an implantable aortic valve device 600 with pre-attached sutures 602.

Some other examples of devices contemplated include cardiac management devices, drug pump devices, support slings, wound repair devices for internal and/or external use, monitoring devices, radio frequency identification devices, transponder devices, MRI-compatible implantable - devices, catheter devices, shunt devices, bone support devices, joint replacement devices, allograft devices, organ replacement structures, and communication devices. Such devices will typically incorporate biocompatible material(s) such as extracellular matrix material, synthetic polymer, biological polymer, alloy, or some combination thereof. Those of skill in the art will appreciate that pre-attached sutures within the scope of the present invention may be appropriate for many other medical devices. Likewise, those of skill in the art will appreciate that variations and combinations beyond those expressly illustrated are within the scope of the present invention as set forth in the appended claims.

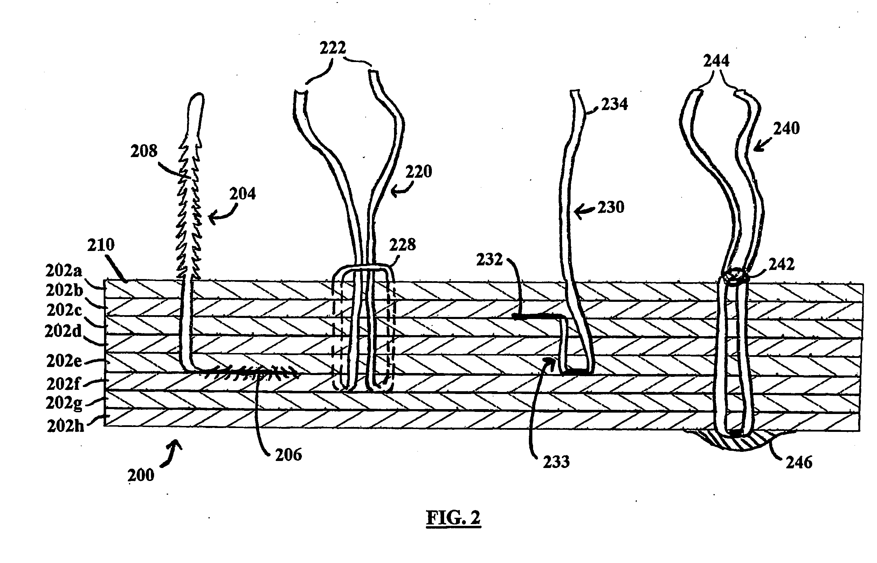

One example of a medical device is illustrated in FIG. 2, which is a longitudinal cross-sectional view (not to scale) of a multilayered medical device embodiment 200 having affixation means embodied as sutures disposed therein. Different types of sutures are shown as well as different examples of how sutures may be attached to the device. While all of the illustrated sutures and attachment configurations could be included in a single device, presently preferred embodiments use a single suture type and mounting configuration.

The medical device 200 preferably includes at least two layers, more preferably at least about five, and yet more preferably at least about eight layers of a biocompatible material 202a-202h. Alternative preferred medical devices can have fewer or additional layers of said biocompatible material. For example, the alternative devices can have 2, 3, 4, 5, 6, 7, 9, 10, 11, 12, 13, 14, 15, 16, 17, 18, 19, 20, or more layers of biocompatible material. Most preferably, the medical device 200 has at least about eight layers of, for example, biocompatible material 202a-202h. Still in a preferred embodiment, a medical device of the present invention may comprise a single layer of suitable biocompatible material. In such an embodiment, the suture or other affixation means preferably is either "patched over" with a suitable material - or is comprised of a material - that resists adhesion, or the single layer of biocompatible material is sufficiently thick that the suture may be secured to it using only a portion of its thickness such that the suture is exposed only on the side it enters (and - if so configured - exits) the layer of material. One such embodiment may include a thick layer of an acellular matrix material, for example such as that which may be derived from dermal tissue.

A number of biocompatible materials suitable for use in a medical device are known in the art, as set forth below. A preferred material is small intestine submucosal material (e.g., Cook SIS(R), manufactured by Cook Biotech, Inc., West Lafayette, Indiana). Collagenous biomaterials, such as small intestinal submucosa, present an advantage over prior art devices using polypropylene and/or other synthetics. In particular, small intestinal submucosa provides a resorbable, substantially acellular matrix material that provides a framework for and is replaced with the patient's own tissue during healing. Accordingly such material leaves little or no foreign material in the patient's body after a time of healing. See, e.g., U.S. Patents 6,206,931 (assigned to Cook, Inc. of Bloomington, Ind., Med Institute, Inc. of W. Lafayette, Ind., and Cook Biotech, Inc. of W. Lafayette, Ind.) and 6,666,892 (assigned to Cook Biotech, Inc.),. Extracellular matrix materials derived from other tissues (e.g., dermis, or other tissue) may also have preferred applications with the present invention. In preferred embodiments, resorbable sutures are used for affixing a medical device to a patient body. Those of skill in the art will appreciate that a variety of polymeric, chromic catgut, or other sutures will be appropriate for use within the scope of the present invention as set forth in the appended claims. Thus, in an embodiment of the presently disclosed medical device using small intestinal submucosa and resorbable sutures, little or no foreign material will remain in a patient at the treatment site following a time of healing wherein the device and sutures are resorbed or otherwise replaced or removed by natural processes. In alternative embodiments, the medical device may include many layers of appropriate biocompatible material(s), currently preferred embodiments include the layers in multiples of two. A presently preferred embodiment includes eight layers as illustrated in FIG. 2. In each of the suture placement configurations, the suture is not exposed on the distal (bottom) surface of the tissue repair device 200, thereby minimizing the likelihood that a suture will form an adhesion or other damaging condition with viscera underlying the tissue comprising the hernia being treated with the device 200.

Non-absorbable biocompatible materials suitable for use with various embodiments of the present invention preferably include the qualities of being mechanically and chemically stable, resistant to degradation, resistant to generation of toxic by-products, and resistant to generation of inflammatory by-products. Some examples of non-absorbable biocompatible materials include tantalum mesh, stainless steel (mesh or other forms), polyester sheeting (Mylar®), polyester cloth (Dacron®), polyester mesh (Mersilene®), polyvinyl cloth (Vinyon-N®), polytetrafluoroethylene mesh or cloth (PTFE/ Teflon®), acrylic cloth (Orlon®) polyvinyl sponge (Ivalon®), expanded PTFE (ePTFE/ GoreTex®), and polypropylene mesh (Marlex® or Prolene®. Some preferred non-absorbable biocompatible materials (e.g., a sufficiently small-pore ePTFE surface) will also resist adhesion or ingrowth by adjacent tissues.

For embodiments of the present invention where cellular incorporation, replacement, and/or some form of resorbability is desired, the biocompatible material should possess mechanical strength. The material should biodegrade in the context of or be replaced by cellular mechanisms such that no toxic or inflammatory by-products are released. Preferably, the bio-removal process by which the biocompatible material is removed/replaced includes or is complemented by cellular mechanisms that effect healing of the condition being treated. Some examples of absorbable biocompatible materials include polyglactin (Vicryl®) and polyglycolic acid (Dexon®). One other biocompatible material, carbon fiber mesh, is not absorbable, but "breaks down such that its fragmented fibers are removed by macrophages. A combination of resorbable or degradable materials may be used in some embodiments as well.

Preferred biocompatible materials do not generate a significant immune response or other cellular response that could cause inflammation or an immune response resulting in rejection of the material. Additionally, preferred biocompatible materials are amenable to anti-microbial treatment such as, for example, heat or chemical sterilization, and/or treatment with one or more antimicrobial materials (e.g., topical placement, impregnation). One specific example of such a material is DualMesh(R) Biomaterial (W. L. Gore & Assoc), which is an ePTFE product. The proximal side (to be placed adjacent to tissue where attachment is desirable) of DualMesh has a greater pore size to enhance tissue incorporation. The distal side (to be placed adjacent to tissue where attachment is not desirable) has a lesser pore size to minimize the likelihood of tissue incorporation or adhesion formation.

The embodiments of medical devices illustrated by the exemplar medical device 200 of FIG. 2 are particularly well-adapted for repair of ventral hernias by the technique of laparoscopic transfascial fixation, which is known in the art of surgical repair (see, e.g., pp. 406-409 of Zollinger's Atlas of Surgical Operations, Eighth Ed.\ R.M. Zollinger, Jr., et al., Eds. 2003, McGraw-Hill Cos., Inc., which illustrates the use of transfascial fixation during repair of a ventral hernia with a prior art patch device). Prior art patch devices typically do not include pre- placed sutures, and a surgeon or attendant may place sutures in the device before applying the device in a patient. The present device includes pre- placed sutures that obviate the need for this extra step, thereby reducing time and handling steps that could compromise the integrity or sterility of the device. Pre-placement of the suture provides for suture placement that will not be exposed on the non-connecting side of a tissue repair or other medical device where exposed sutures may increase the likelihood of adhesion formation. Moreover, those of skill in the art will recognize that a tissue repair or other medical device with pre-attached sutures is also appropriate for use in treating other injuries or lesions.

The first suture is a barbed suture 204. A mounting end 206 of the barbed suture 204 is secured between the fifth and sixth layers 202e, 202f of the medical device 200. The mounting end 206 includes an irregular (e.g., barbed, serrated, and/or crenellated) surface engaging the surrounding layers 202e, 202f (the various irregular surfaces of the suture 204 are hereinafter collectively referred to as "barbed"). Instead of, or in addition to the mounting securement provided by the barbed surface, the mounting end 206 may be secured by an adhesive, by crimping of the surrounding layers 202e, 202f, or another appropriate securing means. In alternative embodiments, the mounting end 206 may be mounted between other layers. An external attachment end 208 extends from the top surface 210 of the medical device 200. The external attachment end 208 also includes a barbed surface for engaging a tissue surface in the region of a patient being treated with the medical device 200. Use of a barbed suture 204 obviates the need to knot the suture because the barbs serve to hold the suture and repair device in place, once the suture is engaged with tissue. Those of skill in the art will appreciate that barbed sutures may be used in other suture placement configurations, within the scope of the present invention. Many different barbed suture configurations are available (see, e.g., U.S. Pat. App. Publ. 2004/144395), and may be suitable for use within the scope of the present invention. Use of barbed sutures may present an advantage in reducing the likelihood of certain undesirable adhesions that may occur between a patent's tissue and the knots of a traditional suture.

The second suture illustrated in FIG. 2 is a chromic catgut resorbable suture 220. The suture 220 is mounted to the medical device 200 in a girth hitch configuration. Specifically, the suture 220 is looped around the top six layers 202a-202f and secured by a girth hitch. See FIG. 2A for a generic illustration showing a perspective view of a girth hitch: to form a girth hitch, both ends 222 of the suture 220 are wrapped about a substrate 226, leaving a loop 228, through which the ends 222 are then directed, thereby securing the suture 220 around the substrate 226.

The third suture illustrated in FIG. 2 is a polyglycolic resorbable suture 230 including two ends 232, 234 with an intermediate portion 233 therebetween. The first end 232 is secured between the second and third layers 202b, 202c of the medical device 200 (e.g., by crimping, adhesive, or another appropriate securing means). The intermediate portion 233 passes down through the third, fourth, and fifth layers 202c-202e, then back up through the fifth-first layers 202e-202a, where the second end extends from the top of the medical device 200. In alternative embodiments, the first end 232 may extend from the top surface 210, and/or it may be tied around the intermediate portion, either on the top surface 210 or between two of the layers 202.

The fourth suture illustrated in FIG. 2 is a polydioxanone resorbable suture 240. The suture 240 is looped through all eight layers 202a-202h and secured to the medical device 200 by a knot 242 (e.g., a commonly used surgical suture knot or other appropriate knot), leaving free the suture ends 244 for attachment to patient tissue. The portion of the suture 240 along the bottom of the eighth layer 202h is covered by a patch layer of a biocompatible material 246 positioned locally over and around the suture 240 and secured to the medical device 200. The biocompatible material may be, for example, PTFE, ePTFE, a biologically-derived matrix (e.g., small intestinal submucosa, dermal-derived matrix), or another appropriate material that is preferably likely to have a lesser risk than an exposed suture surface of forming adhesions to underlying viscera. In some alternative embodiments, the suture 240 need not be knotted and/or may be looped between other layers 202. In another alternative embodiment, the suture 240 is mounted and knotted such that only one free suture end extends from the top surface 210. In another embodiment, the portion of the suture 240 (or other affixation means) on the distal side of the medical device 200 is not covered by the patch layer 246. In such an embodiment, the suture 240 preferably is made of a material with a low likelihood of adhesion formation (e.g., PTFE, ePTFE).

FIG, 3 illustrates a perspective view of a non-equilateral hexagon-shaped tissue repair device embodiment 300 showing one pre-placed suture 302 configuration. The suture placement configuration includes a suture 302 pre-placed near each of the six rounded apices of the device 300. Those of skill in the art will appreciate that a variety of shapes for the device 300 as well as a variety of pre-placed suture configurations will be appropriate for different applications (e.g., repair of different hernia types, treatment/repair of other wound/lesion types), and are within the scope of the present invention as set forth in the appended claims. It should be noted from FIG. 3 that the sutures 302 extending from the top side 304 of the tissue repair device 300 are not exposed on the bottom side 306.

In preferred embodiments, a biocompatible material comprising the device of the present invention also includes glycosaminoglycans, glycoproteins, proteoglycans, and/or one or more growth factors or therapeutic factors on at least a portion of the proximal surface. These growth or therapeutic factors may enhance healing of patient tissue in the region being treated with the device. The therapeutic factors may include, for example, naturally occurring or synthetic compounds known to promote beneficent conditions such as enhanced healing, prevention of infection or inflammation, or repression of tumor or scar tissues. Certain embodiments of the device may be shaped to aid orientation during placement of the device in a patient and/or to aid in treatment of specifically shaped lesions (e.g., the tissue repair device 300 in FIG. 3 is a hexagon). In some embodiments, one or more portions of the suture(s) and/or the device may include visual and/or radio-opaque marking indicia printed, affixed, or otherwise disposed thereon, preferably useful for orientation of the device during placement into a patient. Those of skill in the art will appreciate that many such device shape and/or indicia alternatives are suited for use within the scope of the present invention as set forth in the appended claims. Those of skill in the art will also appreciate that, for devices which are placed using optical visualization (e.g., a laparoscopically placed hernia repair device), visual indicia are preferable over radio-opaque indicia.

There is proposed s a method of repairing a hernia, while decreasing risk of adhesion formation between sutures and underlying tissue/viscera. The method includes a series of steps, known in the art for the procedure of transfascial fixation. A patient is first prepared for a laparoscopic (or open) surgical procedure. (The method is presently related in a laparoscopic surgery context, but those of skill in the art will appreciate that the method may be adapted to a more open surgical procedure without departing from the scope of the present invention). A device of the present invention (e.g., the tissue repair device 300 of FIG. 3) is introduced into a patient through a laparoscopic port. A plurality of sutures is disposed in the biocompatible material sheet comprising the device so as to protrude through the first side of the sheet in a predetermined pattern without being substantially exposed on the second side. The device is deployed across a hernia inside the patient, with the first side oriented toward a tissue region comprising the hernia and the second side oriented toward underlying tissue/viscera. A pattern of small incisions are made on an exterior surface on the patient's body over a location of the hernia, with the pattern of incisions corresponding to the predetermined pattern of sutures on the device. A tool such as a suture passer is directed into at least one of the small incisions and used to grasp or otherwise capture and direct the suture, pulling it so as to bring the device in contact with the tissue region comprising the hernia. Then, the suture is manipulated so as to hold a portion of the device in contact with the tissue region comprising the hernia (e.g., if a barbed suture is used, it is drawn through the incision thereby engaging the appropriate tissue; if a standard suture is used, it is affixed with a knot or other means of securement).

It is intended that the foregoing detailed description be regarded as illustrative rather than limiting, and that it be understood that it is the following claims, including all equivalents, that are intended to define the scope of this invention.

## Claims

1. A medical device, comprising:
a multilayered sheet of biocompatible material, the sheet having a proximal side and a distal side; and
at least one suture having two ends, one end protruding from the proximal side, and the other of the ends being attached to the biocompatible material between layers of the multilayered sheet without the suture being exposed on the distal side.

2. The medical device of claim 1, wherein at least one of the biocompatible material and the suture comprises a resorbable material.

3. The medical device of claim 1, wherein the biocompatible material comprises an extracellular matrix material, preferably derived from small intestinal submucosa.

4. The medical device of claim 3, wherein the sheet is attached to a mounting surface by more than one suture such that at least a portion of the proximal side is in contact with the mounting surface.

5. The medical device of claim 1, wherein the suture comprises an irregular surface selected from the group consisting of barbs, serrations, crennelations, and a combination thereof.

6. The medical device of claim 1, further comprising at least one of a growth factor or a therapeutic factor.

7. The device of claim 1, wherein the device is selected from a group consisting of cardiac management devices, drug pump devices, support slings, wound repair devices for internal use, wound repair devices for external use, monitoring devices, radio frequency identification devices, transponder devices, MRI-compatible implantable devices, catheter devices, shunt devices, bone support devices, joint replacement devices, allograft devices, organ replacement structures, and communication devices.

8. A method of making a medical device according to claim 1, comprising the steps of:
providing one or more first sheets of a first biocompatible material, wherein the one or more first sheets have a proximal side and a distal side;
assembling to the sheet a plurality of sutures, each suture having a first end and a second end, such that at least one of the first and second end of each suture is introduced from the distal side to protrude through the proximal side of the first sheet; and
covering any portion of the plurality of sutures that is exposed on or at the distal side with a second biocompatible material thereby forming a final medical device; wherein the first biocompatible material and the second biocompatible material can be the same or different.

9. The method of claim 8, wherein the covering step comprises:
attaching the second biocompatible material to the distal side of the one or more first sheets, thereby sandwiching the portions of the plurality of sutures exposed on or at the distal side of the one or more first sheets between the one or more first sheets and the second biocompatible material, such that the plurality of sutures is not exposed in the distal side of the final medical device.

10. The method of claim 8, wherein the second biocompatible material is formed into one or more layers thereof prior to the attaching step.

11. The method of claim 8, wherein the first end of at least one of the plurality of sutures is disposed through the one or more first sheets and the second end is disposed between the one or more first sheets and the one or more second sheets.

12. The method of claim 8, wherein both of the first and second ends of at least one of the plurality of sutures are disposed through the one or more first sheets and at least an intervening portion between the first and second end is disposed between the one or more first sheets and the one or more second sheets.

## Patentansprüche

1. Medizinprodukt, das Folgendes umfasst:
eine mehrschichtige Platte aus biokompatiblem Material, wobei die Platte eine proximale Seite und eine distale Seite aufweist; und
zumindest ein Nahtmaterial mit zwei Enden, wobei ein Ende aus der proximalen Seite vorsteht und das andere Ende an dem biokompatiblen Material zwischen Schichten der mehrschichtigen Platte befestigt ist, ohne dass das Nahtmaterial auf der distalen Seite freiliegt.

2. Medizinprodukt nach Anspruch 1, worin zumindest das biokompatible Material und/oder das Nahtmaterial ein resorbierbares Material umfasst.

3. Medizinprodukt nach Anspruch 1, worin das biokompatible Material ein extrazelluläres Matrixmaterial umfasst, das vorzugsweise von Dünndarm-Submukosa stammt.

4. Medizinprodukt nach Anspruch 3, worin die Platte mit mehr als einem Nahtmaterial an einer Befestigungsfläche befestigt ist, so dass zumindest ein Teil der proximalen Seite mit der Befestigungsfläche in Berührung steht.

5. Medizinprodukt nach Anspruch 1, worin das Nahtmaterial eine unregelmäßige Oberfläche aufweist, die aus der Widerhaken, Verzahnungen, Krenelierungen und eine Kombination davon umfassenden Gruppe ausgewählt ist.

6. Medizinprodukt nach Anspruch 1, das ferner zumindest einen Wachstumsfaktor und/oder einen therapeutischen Faktor umfasst.

7. Medizinprodukt nach Anspruch 1, worin das Produkt aus einer Herzmanagementvorrichtungen, Arzneimittelpumpenvorrichtungen, Stützschlingen, Wundreparaturvorrichtungen für den inneren Gebrauch, Wundreparaturvorrichtungen für den äußeren Gebrauch, Überwachungsvorrichtungen, Hochfrequenz-Identifikationsvorrichtungen, Transpondervorrichtungen, MRT-kompatible implantierbare Vorrichtungen, Kathetervorrichtungen, Shuntvorrichtungen, Knochenstützvorrichtungen, Gelenkersatzvorrichtungen, Allotransplantatvorrichtungen, Organersatzkonstruktionen und Kommunikationsvorrichtungen umfassenden Gruppe ausgewählt ist.

8. Verfahren zur Herstellung eines Medizinprodukts nach Anspruch 1, das die folgenden Schritte umfasst:
Bereitstellung von ein oder mehr ersten Platten aus einem ersten biokompatiblen Material, worin die ein oder mehr ersten Platten eine proximale Seite und eine distale Seite aufweisen;
Anordnung einer Vielzahl von Nahtmaterialien auf der Platte, wobei jedes Nahtmaterial ein erstes Ende und ein zweites Ende aufweist, so dass zumindest das erste Ende und/oder das zweite Ende jedes Nahtmaterials von der distalen Seite eingeführt wird, um durch die proximale Seite der ersten Platte vorzustehen; und
Abdeckung eines beliebigen Teils der Vielzahl von Nahtmaterialien, der auf oder an der distalen Seite freiliegt, mit einem zweiten biokompatiblen Material, wodurch ein endgültiges Medizinprodukt geformt wird; worin das erste biokompatible Material und das zweite biokompatible Material gleich oder verschieden sein können.

9. Verfahren nach Anspruch 8, worin der Abdeckschritt Folgendes umfasst:
Befestigung des zweiten biokompatiblen Materials auf der distalen Seite der ein oder mehr ersten Platten, so dass die Teile der Vielzahl von Nahtmaterialien, die auf oder an der distalen Seite der ein oder mehr ersten Platten freiliegen sandwichartig zwischen den ein oder mehr ersten Platten und dem zweiten biokompatiblen Material aufgenommen werden, so dass die Vielzahl von Nahtmaterialien in der distalen Seite des endgültigen Medizinprodukts nicht mehr freiliegt.

10. Verfahren nach Anspruch 8, worin das zweite biokompatible Material vor dem Befestigungsschritt zu ein oder mehr Schichten geformt wird.

11. Verfahren nach Anspruch 8, worin das erste Ende zumindest eines der Vielzahl von Nahtmaterialien durch die ein oder mehr ersten Platten angeordnet wird und das zweite Ende zwischen den ein oder mehr ersten Platten und den ein oder mehr zweiten Platten angeordnet wird.

12. Verfahren nach Anspruch 8, worin die ersten und zweiten Enden zumindest eines der Vielzahl von Nahtmaterialien beide durch die ein oder mehr ersten Platten angeordnet sind und zumindest ein eingreifender Abschnitt zwischen dem ersten und zweiten Ende zwischen den ein oder mehr ersten Platten und den ein oder mehr zweiten Platten angeordnet ist.

## Revendications

1. Dispositif médical, comportant :
une feuille multicouche de matériau biocompatible, la feuille présentant une face proximale et une face distale ; et
au moins une suture présentant deux extrémités, une extrémité faisant saillie depuis la face proximale, et l'autre extrémité étant fixée au matériau biocompatible entre les couches de la feuille multicouche sans que la suture soit exposée sur la face distale.

2. Dispositif médical selon la revendication 1, dans lequel au moins le matériau biocompatible et/ou la suture comporte un matériau résorbable.

3. Dispositif médical selon la revendication 1, dans lequel le matériau biocompatible comporte un matériau de matrice extracellulaire, de préférence issu de sous-muqueuse d'intestin grêle.

4. Dispositif médical selon la revendication 3, dans lequel la feuille est fixée à une surface de montage par plusieurs sutures de sorte qu'au moins une portion de la face proximale est en contact avec la surface de montage.

5. Dispositif médical selon la revendication 1, dans lequel la suture comporte une surface irrégulière choisie dans le groupe constitué d'ardillons, de dentelures, de crénelures, et une combinaison de ceux-ci.

6. Dispositif médical selon la revendication 1, comportant en outre au moins un facteur de croissance ou un facteur thérapeutique.

7. Dispositif selon la revendication 1, dans lequel le dispositif est choisi dans un groupe constitué de dispositifs de gestion cardiaque, de dispositifs à pompe à médicaments, de bandages de soutien, de dispositifs de cicatrisation des blessures à usage interne, de dispositifs de cicatrisation des blessures à usage externe, de dispositifs de surveillance, de dispositifs d'identification par radiofréquence, de dispositifs transpondeurs, de dispositifs implantables compatibles avec l'IRM, de dispositifs à cathéter, de dispositifs de dérivation, de dispositifs de support d'os, de dispositifs de remplacement d'articulation, de dispositifs d'allogreffe, de structures de remplacement d'organe, et de dispositifs de communication.

8. Procédé pour fabriquer un dispositif médical selon la revendication 1, comportant les étapes suivantes :
fournir une ou plusieurs premières feuilles d'un premier matériau biocompatible, dans lequel lesdites une ou plusieurs premières feuilles présentent une face proximale et une face distale ;
monter sur la feuille une pluralité de sutures, chaque suture présentant une première extrémité et une deuxième extrémité, de sorte qu'au moins une desdites première et deuxième extrémités de chaque suture est introduite depuis la face distale pour faire saillie à travers la face proximale de la première feuille ; et
recouvrir une portion quelconque de ladite pluralité de sutures qui est exposée sur la face distale ou au niveau de celle-ci d'un deuxième matériau biocompatible formant ainsi un dispositif médical final ;
dans lequel le premier matériau biocompatible et le deuxième matériau biocompatible peuvent être analogues ou différents.

9. Procédé selon la revendication 8, dans lequel l'étape consistant à recouvrir comporte les étapes suivantes :
fixer le deuxième matériau biocompatible à la face distale desdites une ou plusieurs premières feuilles, en intercalant ainsi les portions de la pluralité de sutures exposées sur la face distale, ou au niveau de celle-ci, desdites une ou plusieurs premières feuilles entre lesdites une ou plusieurs premières feuilles et le deuxième matériau biocompatible, de sorte que la pluralité de sutures n'est pas exposée dans la face distale du dispositif médical final.

10. Procédé selon la revendication 8, dans lequel le deuxième matériau biocompatible est formé en une ou plusieurs couches de celui-ci avant l'étape consistant à fixer.

11. Procédé selon la revendication 8, dans lequel la première extrémité d'au moins une suture de ladite pluralité de sutures est disposée à travers lesdites une ou plusieurs premières feuilles et la deuxième extrémité est disposée entre lesdites une ou plusieurs premières feuilles et lesdites une ou plusieurs deuxièmes feuilles.

12. Procédé selon la revendication 8, dans lequel lesdites première et deuxième extrémités d'au moins une suture de ladite pluralité de sutures sont toutes les deux disposées à travers lesdites une ou plusieurs premières feuilles et au moins une portion intermédiaire entre les première et deuxième extrémités est disposée entre lesdites une ou plusieurs premières feuilles et lesdites une ou plusieurs deuxièmes feuilles.
